# EUROPEAN PATENT APPLICATION

(11) **EP 1 949 845 A2**
(43) Date of publication of application: **30.07.2008**
(21) Application number: 07013526.4
(22) Date of filing: 10.07.2007
(51) Int. Cl.: A61B 1/00

(54) **Operation control system and operation control method**

(30) Priority: 21.07.2006 JP 2006199974
(71) Applicant: OLYMPUS MEDICAL SYSTEMS CORP., Shibuya-ku Tokyo 151-0072 (JP)
(72) Inventor: Koishi Tashiro c/o Olympus Medical Systems Corp., Tokyo 151-0072 (JP); Uchikubo, Akinobu c/o Olympus Medical Systems Corp., Tokyo 151-0072 (JP); Nakamura, Takeaki c/o Olympus Medical Systems Corp., Tokyo 151-0072 (JP)
(74) Representative: von Hellfeld, Axel

(57) **Abstract**

According to the invention, an infrared communication port of a system controller performs infrared communication with a mobile remote controller, which allows function assignment to the mobile remote controller. The mobile remote controller requests controllable appliance list data to the system controller in response to an operation on a key input select button in a fixed key display area. Then, the mobile remote controller receives the controllable appliance list data, and an appliance operation button is assigned to a function key display area. Thus, multiple controllable appliances can be controlled easily through one mobile remote controller by allowing assigning control functions for the multiple controllable appliances easily to the mobile remote controller.

## Description

### BACKGROUND OF THE INVENTION

### 1. Field of the Invention

The present invention relates to an operation control system including a medical system and medical support system and operation control method.

### 2. Description of the Related Art

In recent years, an endoscopic operation system that performs a technique by using an endoscope has been widely spread, and wide varieties of medical appliance are used therein.

Medical appliances used in the endoscopic operation system include an electric knife device, an ultrasonic device and an insufflator in addition to an electronic endoscopic system. As disclosed in Japanese Unexamined Patent Application Publication No. 2003-76786 or 2003-70746, for example, these appliances are centrally managed in a system and are controlled by an operation device deployed under a system controller.

On the other hand, various peripheral appliances in addition to the medical appliances above are placed in an operation room having a medical system, typified by the endoscopic operation system. For example, the peripheral appliances may include a server that stores a reference image such as a CT image, and an ultrasonic image before an operation of a patient, a display device that displays the reference image, which is referred for a technique, an output device that records or prints an endoscopic image picked up by the endoscopic operation system, a video conference system that communicates with the outside of the operation room, and a room light that controls the illumination in the operation room.

For the variety of peripheral appliances, an AV system is established, which is a non-medical appliance system separately provided from a system that controls a medical appliance and is controlled by a different operation device from the operation device for controlling a medical appliance.

A general consumer appliance such as a VTR, a DVD and a liquid crystal monitor may be included in the peripheral appliances in the AV system. Such a consumer appliance generally comes with a mobile remote controller.

In recent years, general consumer-product sections have developed a remote controller with a learning function (called learning remote controller below) by which a necessary function of a mobile remote controller for each consumer appliance can be assigned to a function key by using an infrared communication function, for example, since each consumer appliance has a mobile remote controller.

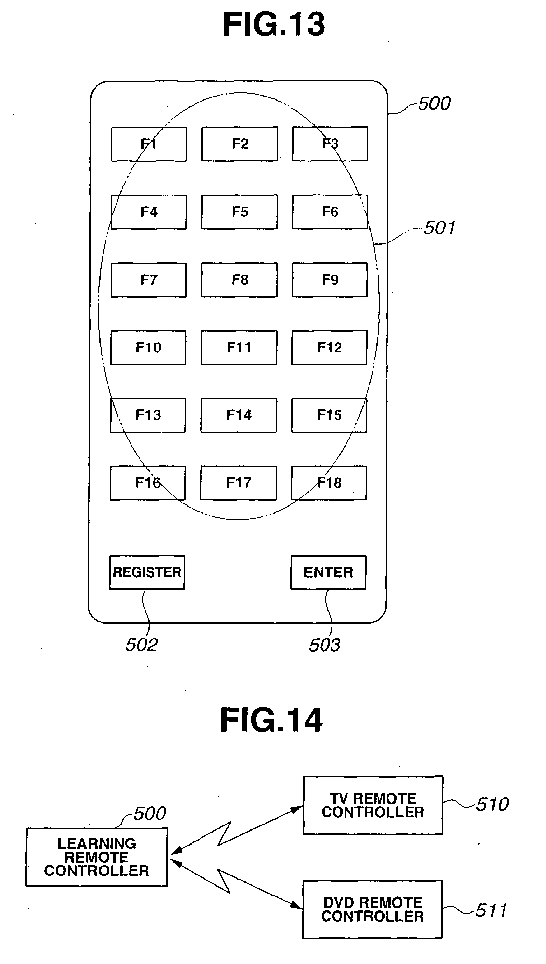

Fig. 13 shows an example of the appearance of the learning remote controller. As shown in Fig. 13, a learning remote controller 500 includes a function key section 501, a select button 502 and an enter button 503. The function key section 501 has multiple function keys to which functions can be assigned. The select button 502 is used to select a function key in the function key section 501. The enter button 503 is used for determining the function assignment to a selected function key.

As shown in Fig. 14, the learning remote controller 500 is faced toward multiple appliance remote controllers the functions of which are required to assign to the learning remote controller 500, such as a TV remote controller 510 and a DVD remote controller 511, and desired key functions of the TV remote controller 510 and DVD remote controller 511 are assigned to function keys selected through the select button 502 by infrared communication. Then, the function assignments can be determined by the enter button 503.

However, the learning remote controller 500 requires the function assignment to each function key. An AV system used with an endoscopic operation system connects to a different peripheral appliance for each technique, and a different control function is required for each peripheral appliance to be controlled.

Therefore, a process for assigning functions of peripheral appliances of an AV system used with an endoscopic operation system to the learning remote controller 500 requires selecting a necessary function from each of remote controllers for the appliances and assigning the function to each function key and further requires changing the function assignment for each technique. This significantly complicates the function assignment process and currently makes the control over the peripheral appliances of the AV system by using the learning remote controller 500 difficult.

### SUMMARY OF THE INVENTION

Accordingly, the invention was made in view of these points, and it is an object of the present invention to provide an operation control system that can control multiple controllable appliances easily through one mobile remote controller by allowing assigning control functions for the multiple controllable appliances easily to the mobile remote controller.

According to the present invention, there is provided an operation control system including:
a first system controller that controls a first controllable appliance to be used for performing a predetermined technique;
a second system controller that controls a second controllable appliance to be used for performing the predetermined technique;
a mobile controller with which a function for operating the second controllable appliance can be registered at least; and
registration information creating means for creating registration information of a function to be registered with the mobile controller based on the second controllable appliance connecting to the second system controller.

The other features and advantages of the present invention will be sufficiently apparent from following descriptions.

### BRIEF DESCRIPTION OF THE DRAWINGS

Fig. 1 is a configuration diagram showing a configuration of an endoscopic operation system according to a first embodiment of the present invention;
Fig. 2 is a block diagram showing a configuration of an operation room control system having the endoscopic operation system and AV system in Fig. 1 according to the first embodiment;
Fig. 3 is a view of the operation room control system in Fig. 2 from the upper part of the operation room according to the first embodiment;
Fig. 4 is a block diagram showing a configuration of the mobile remote controller in Fig. 2 according to the first embodiment;
Fig. 5 is a diagram showing an appearance of the mobile remote controller in Fig. 4 according to the first embodiment;
Fig. 6 is a flowchart showing a flow of processing of assigning an appliance operation button on the mobile remote controller in Fig. 4 according to the first embodiment;
Fig. 7 is a diagram showing a first example of a controllable appliance list table created by the system controller in Fig. 2 according to the first embodiment;
Fig. 8 is a diagram showing a second example of a controllable appliance list table created by the system controller in Fig. 2 according to the first embodiment;
Fig. 9 is a diagram showing an assignment example of an appliance operation button on a mobile remote controller based on the controllable appliance list table in Fig. 7 according to the first embodiment;
Fig. 10 is a diagram showing an example of a changed layout of assignments on the appliance operation buttons in Fig. 9 according to the first embodiment;
Fig. 11 is a diagram showing an assignment example on the appliance operation buttons on the mobile remote controller based on the controllable appliance list table in Fig. 8 according to the first embodiment;
Fig. 12 is a diagram showing an assignment example on the appliance operation buttons on the mobile remote controller in which the operation buttons of the controllable appliances by the system controller in Fig. 2 are assigned thereto according to the first embodiment;
Fig. 13 is a diagram showing the appearance of a conventional learning remote controller; and
Fig. 14 is a diagram illustrating the registration of a function on the learning remote controller in Fig. 13.

### DETAILED DESCRIPTION OF THE PREFERRED EMBODIMENTS

### [First Embodiment]

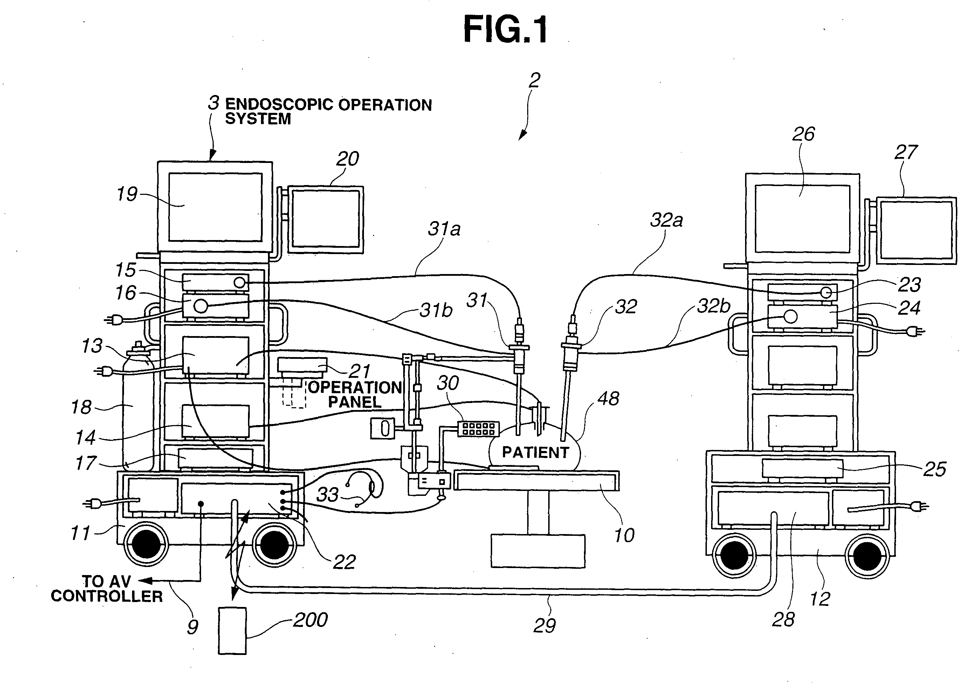

With reference to Fig. 1, an entire configuration of an endoscopic operation system 3 functioning as a medical system placed in an operation room 2 will be described first.

As shown in Fig. 1, a patient bed 10 on which a patient 48 lies down and the endoscopic operation system 3 are placed within the operation room 2. The endoscopic operation system 3 has a first cart 11 and a second cart 12.

The first cart 11 may have, for example, devices such as an electric knife device 13, an insufflator 14, an endoscope camera device 15, a light source device 16 and a video tape recorder (VTR) 17 and a gas cylinder 18 filled with carbon dioxide, which are medical appliances to be controlled. The endoscope camera device 15 is connected to a first endoscope 31 via a camera cable 31 a. The light source device 16 is connected to the first endoscope 31 via a light guide cable 31b.

The first cart 11 further has a display device 19, a first central display panel 20 including a liquid crystal display (LCD), for example, an operation panel 21 functioning as first touch panel means. The display device 19 may display an endoscopic image, such as a TV monitor.

The central display panel 20 functions as display means that can selectively display any data during an operation. The operation panel 21 includes a display section such as a liquid crystal display and a touch panel, for example, integrated onto the display section and functions as a central operation device to be operated by a nurse or the like in a non-clean area for controlling the medical appliances in the endoscopic operation system 3.

The first cart 11 further has a system controller 22, which functions as a first system controller. The system controller 22 is connected to the electric knife device 13, insufflator 14, endoscope camera device 15, light source device 16 and VTR 17 through a communication line, not shown. A head-set type microcomputer 33 can be connected to the system controller 22. The system controller 22 recognizes voice inputted from the microcomputer 33 and can control each appliance by voice of a surgeon.

On the other hand, the second cart 12 has an endoscope camera device 23, a light source device 24, an image processing device 25, a display device 26 and a second central display panel 27 including a liquid crystal display (LCD), which are to be controlled.

The endoscope camera device 23 is connected to a second endoscope 32 via a camera cable 32a. The light source device 24 is connected to the second endoscope 32 via a light guide cable 32b.

The display device 26 may display an endoscopic image captured by the endoscope camera device 23. The second central display panel 27 can selectively display any data during an operation.

The endoscope camera device 23, light source device 24 and image processing section 25 are connected to a relay unit 28 in the second cart 12 through a communication line, not shown. Then, the relay unit 28 is connected to the system controller 22 in the first cart 11 through a relay cable 29.

Thus, the system controller 22 can centrally control the camera device 23, light source device 24 and image processing device 25 in the second cart 12 and the electric knife device 13, insufflator 14, camera device 15, light source device 16 and VTR 17 in the first cart 11. Therefore, when the system controller 22 communicates with these devices, the system controller 22 can display a setting state of the connected appliances and/or a setting screen for an operation switch, for example, on the liquid crystal display of the operation panel 21 above. The system controller 22 allows an operation input for changing a set value, for example, by touching a desired operation switch on the operation panel 21 and operating a touch panel in a predetermined area.

The remote controller 30 is a second central operation device to be operated by a surgeon in a disinfected area and allows other devices the connection with which is established to be operated through the system controller 22.

The system controller 22 is connected to a patient monitor system, not shown, and can analyze biological information obtained from the patient monitor system and display the analysis result to a desired display device.

An infrared communication port (not shown), which functions as communication means, is mounted on the system controller 22. The infrared communication port performs infrared communication with a mobile remote controller 200 for appliances, which allows function assignment to the mobile remote controller 200. The mobile remote controller 200 can be connected to the system controller 22 via a cable.

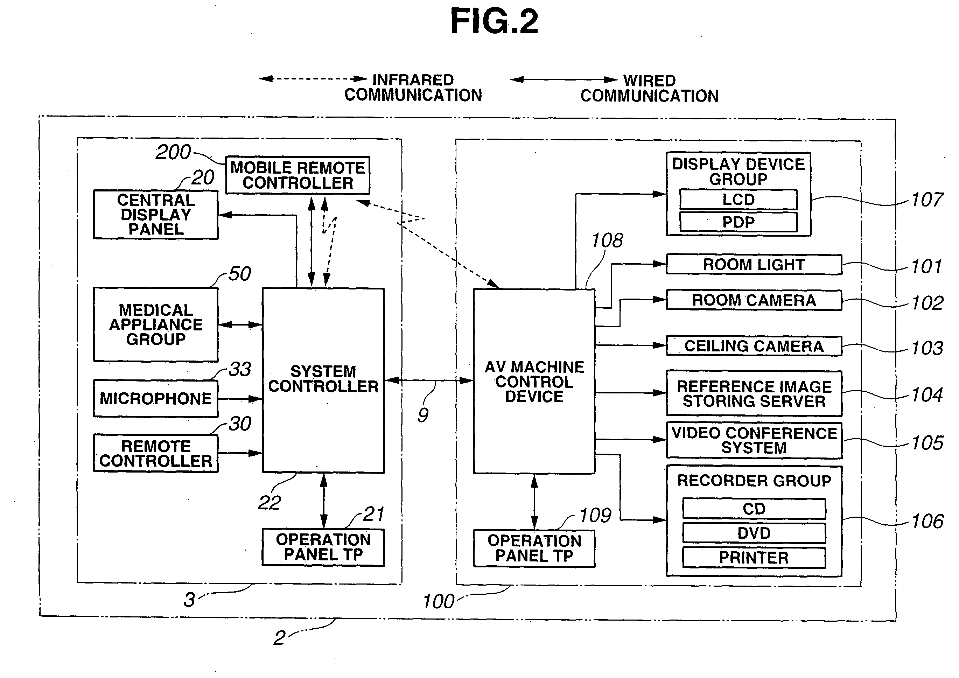

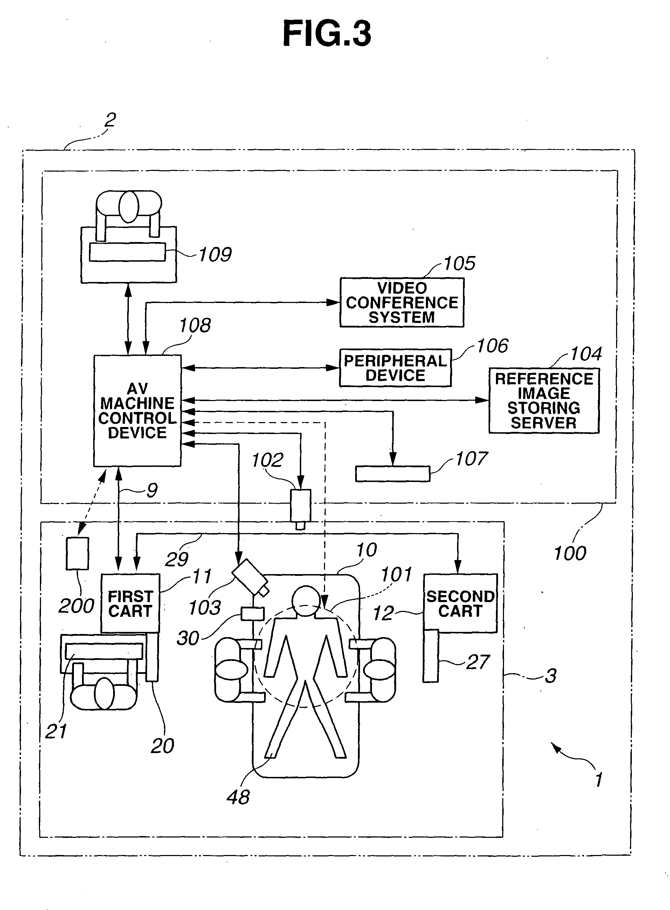

As shown in Figs. 2 and 3, the operation room 2 has an operation room control system having the endoscopic operation system 3. The operation room control system includes the endoscopic operation system 3 functioning as a medical system including the central display panel 20, operation panel 21, system controller 22, microphone 33, remote controller 30 and a medical appliance group 50 including the light source device 24 and an AV system 100 having various audio visual appliances and illumination appliances.

The AV system 100 includes a room light 101, a room camera 102, a ceiling camera 103, a reference image storing server 104, a video conference system 105, a peripheral device 106, a display device 107, an AV appliance control device 108, which is a second system controller that controls these devices and an operation panel 109. The operation panel 109 may include a display section such as a liquid crystal display and a touch panel integrated onto the display section, for example. The operation panel 109 functions as a central operation device to be operated by a nurse or the like in a non-clean area for controlling a device in the system 100.

The reference images storing server 104 stores a CT image and/or ultrasonic image of the patient 48 before an operation, and these images can be displayed on an LCD or PDP in the display device 107.

The AV appliance control device 108 can exchange information with the system controller 22 in the endoscopic operation system 3 via a signal cable 9. The AV appliance control device 108 may display an endoscopic image from the endoscopic operation system 3 on the display device 107 and output the endoscopic image to a CD, DVD or printer in the peripheral device 106.

The AV appliance control device 108 allows an operation input for changing a set value, for example, by touching a desired operation switch on the operation panel 109 (with a touch panel function) and operating a touch panel in a predetermined area.

The information on an appliance, which is connected to the AV appliance control device 108 and is controllable by the AV appliance control device 108, is transmitted to the system controller 22 in the endoscopic operation system 3 via the signal cable 9. The system controller 22 creates a controllable appliance list table, which will be described later, to be assigned to the mobile remote controller 200 based on information on appliances controllable by the AV appliance control device 108.

An infrared communication port (not shown), which functions as communication means, is mounted on the AV appliance control device 108. The infrared communication port can perform infrared communication with a mobile remote controller 200 and controls an appliance connecting to the AV appliance control device 108 by an operation on the mobile remote controller 200.

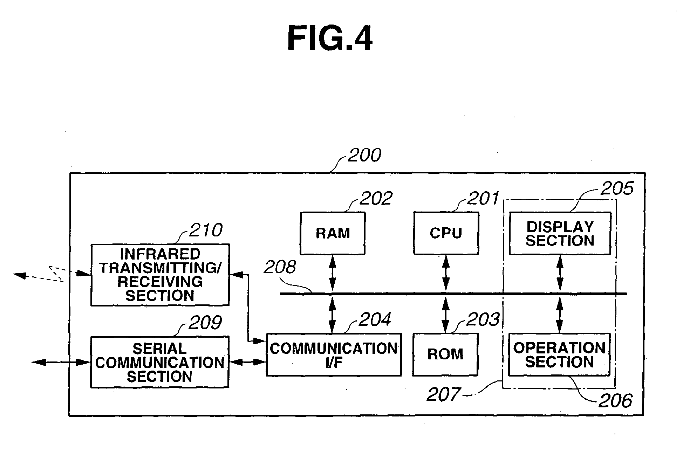

As shown in Fig. 4, the mobile remote controller 200 includes a CPU 201, a RAM 202, a ROM 203, a communication I/F section 204, a display device 205 and an operation section 206 connected through an internal bus 208. A touch panel section 207 includes the display section 205 and operation section 206. The communication I/F section 204 is connected to a serial communication section 209 and an infrared communication section 210 and is configured to be communicable with the outside through the serial communication section 209 and infrared communication section 210.

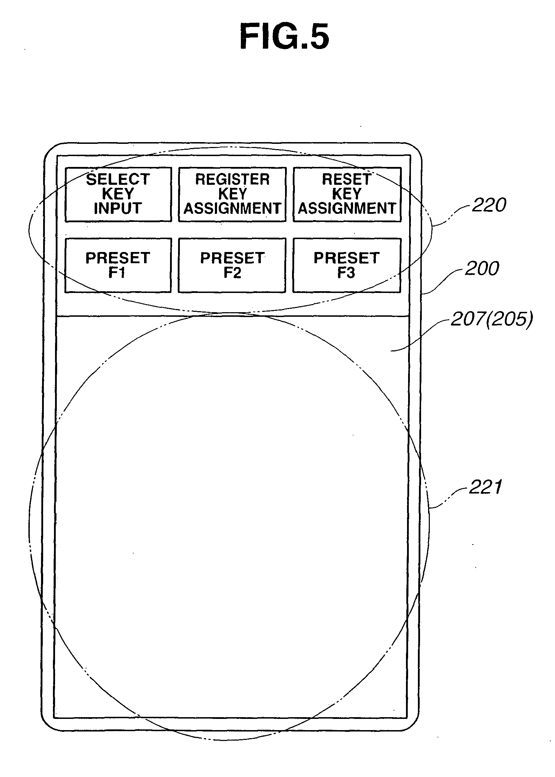

The touch panel section 207 in the mobile remote controller 200 includes, as shown in Fig. 5, a fixed key display area 220 and an assignment function key display area 221 to which a desired function is assigned.

Operations of an operation room control system according to this embodiment, which includes the endoscopic operation system 3 and AV system 100 thus configured, will be described.

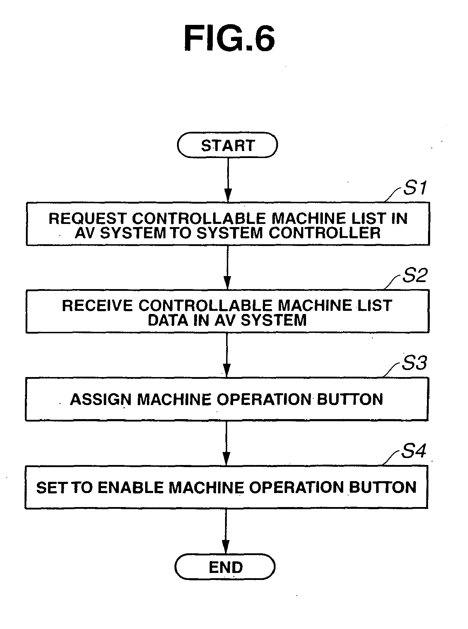

According to this embodiment, the assignment of a desired appliance operation button to the assignment function key display area 221 in the touch panel section 207 on the mobile remote controller 200 through infrared communication (or wired communication) between the system controller 22 in the endoscopic operation system 3 and the mobile remote controller 200 is performed before a technique as illustrated in the flowchart shown in Fig. 6.

In other words, when communication is established between the system controller 22 and the mobile remote controller 200, the mobile remote controller 200 requests controllable appliance list data in the AV system 100 to the system controller 22 in step S1, as shown in Fig. 6.

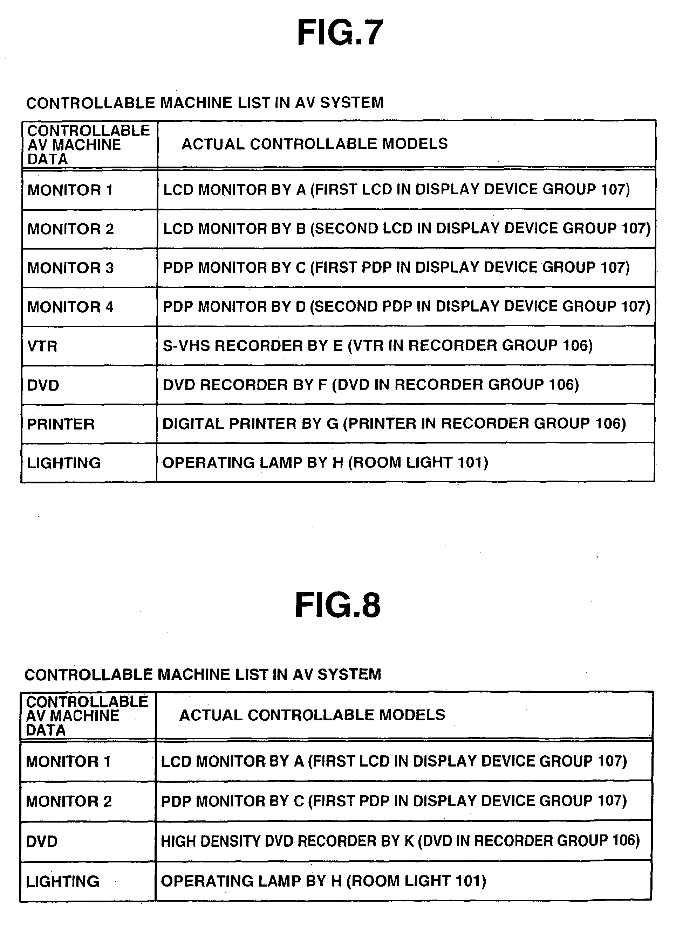

More specifically, the system controller 22 has a CPU (not shown), which functions as registration information creating means having a registration information creating function. The system controller 22 obtains information on an appliance, which is controllable by the AV appliance control device 108, through the AV appliance control device 108 of the AV system 100 and via the signal cable 9. The system controller 22 then creates a controllable appliance list table, as shown in Fig. 7, to be controlled by the mobile remote controller 200. For example, the system controller 22 creates a controllable appliance list table having controllable AV appliance data as shown in Fig. 7 in accordance with the selection of a controllable appliance to be controlled by the mobile remote controller 200 of appliances controllable by the AV machine control device 108 through the operation panel 21 based on a technique.

Fig. 7 shows an example in which a controllable appliance list table is created in accordance with the selection of four monitors, a VTR, a DVD, a printer and a lighting device to be used for a first technique as controllable appliances to be controlled by the mobile remote controller 200. Fig. 8 shows an example in which a controllable appliance list table is created in accordance with the selection of two monitors, a DVD and a lighting device to be used for a second technique, which is different from the first technique, as controllable appliances to be controlled by the mobile remote controller 200.

Having described that the system controller 22 recognizes the category of an appliance controllable by the system controller 22 in this embodiment, the system controller 22 does not have to recognize the actual specific model of the appliance. The AV appliance control device 108 receives a control command through infrared communication from the mobile remote controller 200, and the AV appliance control device 108 thus controls in accordance with an actual specific model.

The mobile remote controller 200 receives controllable appliance list data, which is controllable AV appliance data on the controllable appliance list table created in this way, from the system controller 22 in step S2 and assigns an appliance operation button to the assignment function key display area 221 in step S3.

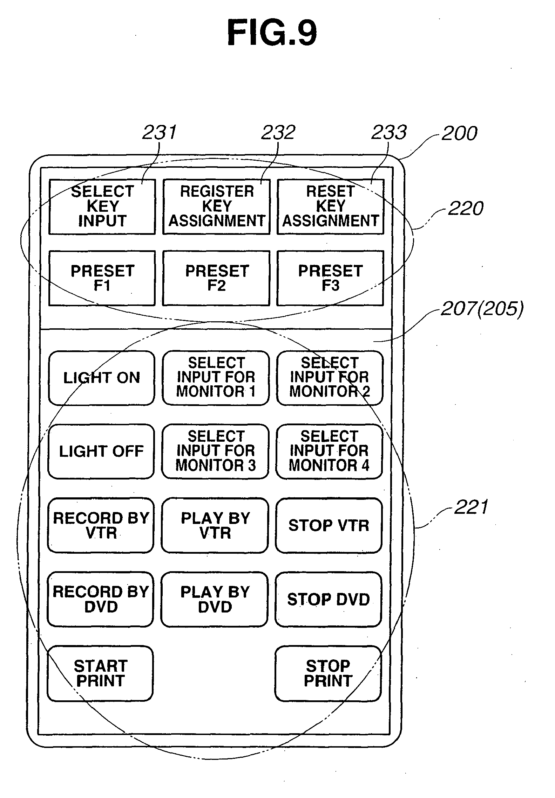

Fig. 9 shows a mobile remote controller 200 resulting from the assignment of appliance operation buttons to the assignment function key display area 221 based on the controllable appliance list table in Fig. 7.

The mobile remote controller 200 requests controllable appliance list data to the system controller 22 in step S 1 above in response to an operation on a key input select button 231 in the fixed key display area 220. Then, the mobile remote controller 200 receives the controllable appliance list data in step S2 above, and an appliance operation button as shown in Fig. 9 is assigned to the function key display area 221 in step S3 above.

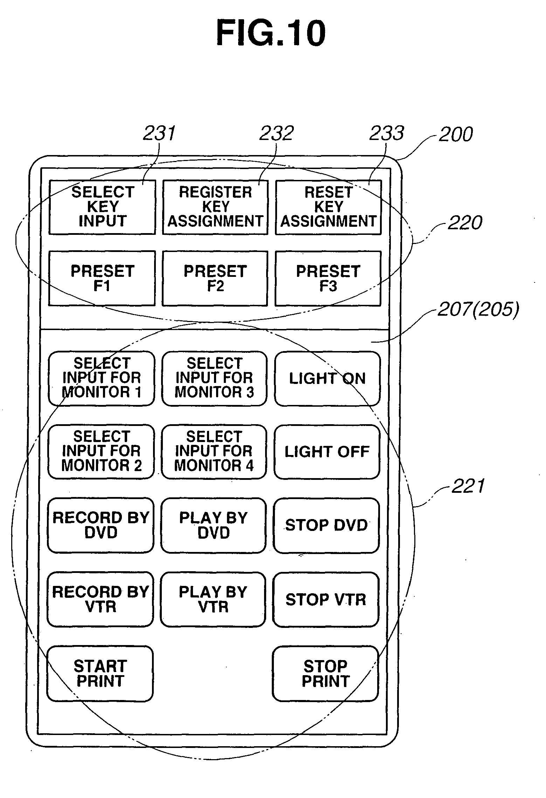

For example, according to this embodiment, assignment layout information on appliance operation buttons in the function key display area 221 may be also transmitted from the system controller 22 to the mobile remote controller 200. Here, in response to an operation on a key assignment reset button 233 in the fixed key display area 220, for example, the mobile remote controller 200 can change the assignment layout of appliance operation buttons to the one as shown in Fig. 10, for example.

Then, the mobile remote controller 200 in step S4 sets to enable the appliance operation buttons in the function key display area 221 in response to an operation on a key assignment registration button 232 in the fixed key display area 220 and exits the processing.

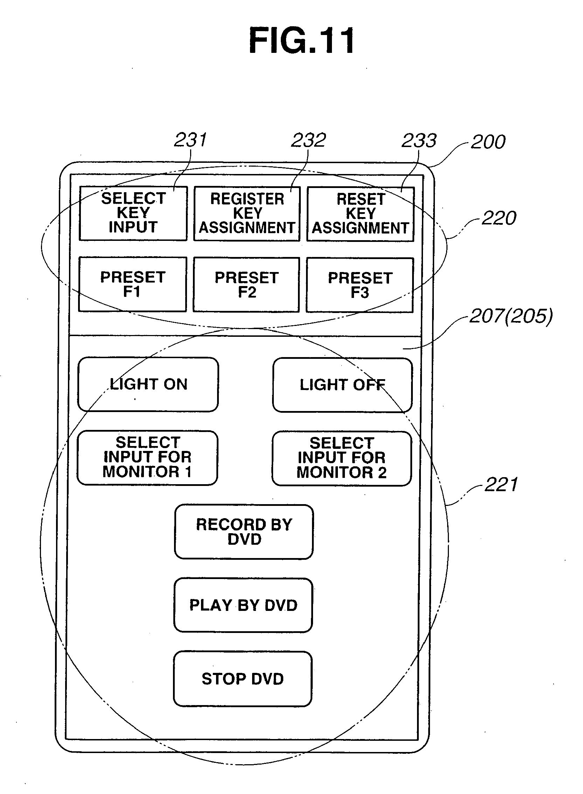

Fig. 11 shows the mobile remote controller 200 resulting from the assignment of appliance operation buttons to the assignment function key display area 221 based on the controllable appliance list table in Fig. 8.

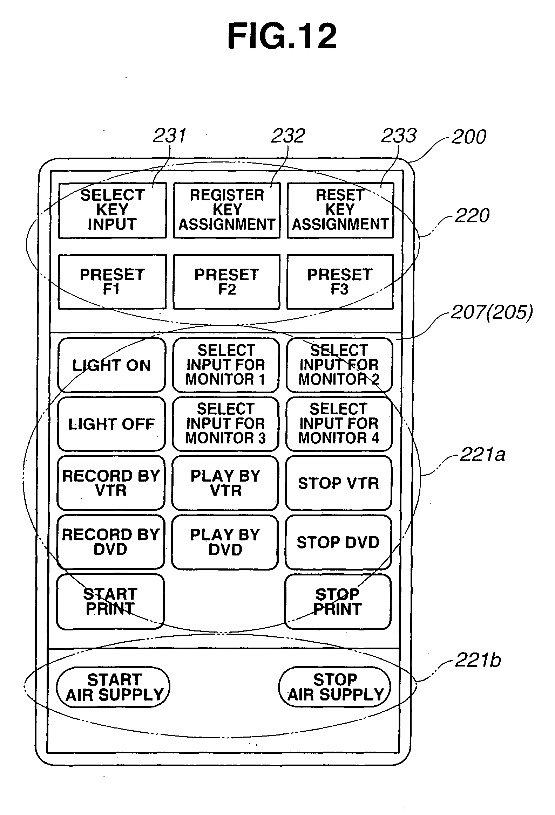

The mobile remote controller 200 may be used to define not only controllable appliances to be controlled by the AV appliance control device 108 but also appliance operation buttons for operating controllable appliances to be controlled by the system controller 22. Fig. 12 shows an example of the mobile remote controller 200 in which respective appliance operation buttons are set in a function key display area 221 a for the AV appliance control device 108 and a function key display area 221b for the system controller 22.

In the example in Fig. 12, the mobile remote controller 200 has settings of assignment of appliance operation buttons based on the controllable appliance list table in Fig. 7 in the function key display area 221a and assignment of operation buttons of the insufflator 14 to be controlled by the system controller 22 in the function key display area 221 b.

In this embodiment, the system controller 22 and AV appliance control device 108 communicate with each other, and the system controller 22 creates a controllable appliance list table from information, on controllable appliances connecting to the AV appliance control device 108. Then, the system controller 22 can define functions operable by the mobile remote controller 200 by only downloading the controllable appliance list data on the controllable appliance list table to the mobile remote controller 200. Therefore, according to this embodiment, the mobile remote controller 200 that allows easily assigning an appliance control function required by a technique and allows operating multiple appliances can be reset for each technique for easy use.

Having described that the present embodiment includes creating a controllable appliance list table by the system controller 22, downloading controllable appliance list data to the mobile remote controller 200 and assigning appliance operation buttons, the present invention is not limited thereto. A controllable appliance list table may be created by the AV appliance control device 108, and controllable appliance list data may be downloaded to the mobile remote controller 200 to assign appliance operation buttons.

It is apparent that the wide variety of embodiments of the present invention can be configured based on the present invention without departing from the spirit and scope of the invention. The present invention is not limited to a specific embodiment thereof except for the appended claims.

## Claims

1. An operation control system comprising:
a first system controller that controls a first controllable appliance to be used for performing a predetermined technique;
a second system controller that controls a second controllable appliance to be used for performing the predetermined technique;
a mobile controller with which a function for operating the second controllable appliance can be registered at least; and
registration information creating means for creating registration information of a function to be registered with the mobile controller based on the second controllable appliance connecting to the second system controller.

2. The operation control system according to Claim 1, wherein the registration information creating means is provided in the first system controller; and
the first system controller downloads registration information to the mobile controller.

3. The operation control system according to Claim 1, wherein the mobile controller operates at least the second controllable appliance by wireless communication.

4. The operation control system according to Claim 2, wherein the mobile controller operates at least the second controllable appliance by wireless communication.

5. An operation control method that controls:
a first system controller that controls a first contrivable appliance to be used for performing a predetermined technique;
a second system controller that controls a second controllable appliance to be used for performing the predetermined technique; and
a mobile controller with which a function for operating the second controllable appliance can be registered at least,
the method comprising:
a registration information creating step of creating registration information of a function to be registered with the mobile controller based on the second controllable appliance connecting to the second system controller.

6. The operation control method according to Claim 5, wherein the registration information creating step is performed in the first system controller; and
the first system controller downloads registration information to the mobile controller.

7. The operation control method according to Claim 5, wherein the mobile controller operates at least the second controllable appliance by wireless communication.

8. The operation control method according to Claim 5, wherein the mobile controller operates at least the second controllable appliance by wireless communication.
